# EUROPÄISCHE PATENTSCHRIFT

(11) **EP 0 639 378 B1**
(45) Veröffentlichungstag und Bekanntmachung des Hinweises auf die Patenterteilung: **08.11.2000**
(21) Anmeldenummer: 94202325.0
(22) Anmeldetag: 16.08.1994
(51) Int. Cl.: A61K 33/00, A61K 9/72, A61M 16/12

(54) **NO-haltiges Gasgemisch zur Behandlung pulmonaler Erkrankungen**
NO-containing gaseous mixture for the treatment of pulmonary diseases
Mélange de gaz contenant du NO pour le traitement de maladies pulmonaires

(30) Priorität: 18.08.1993 DE 4327731
(43) Veröffentlichungstag der Anmeldung: 22.02.1995
(73) Patentinhaber: MESSER GRIESHEIM AUSTRIA Ges.m.b.H., 2352 Gumpoldskirchen (AT)
(72) Erfinder: Krebs, Christian, A-1120 Wien (AT)
(74) Vertreter: Berdux, Klaus, Dipl.-Ing.

(56) Entgegenhaltungen:
- WO-A-88/06904
- WO-A-92/10228
- US-A- 3 676 563
- PATENT ABSTRACTS OF JAPAN, unexamined applications, C Field, Band 7, Nr. 61, 15. M rz 1983 THE PATENT OFFICE JAPANESE GOVERNMENT Seite 164 C 156; & JP-A-57 212 121 (TEISAN K.K.)
- CHEMICAL ABSTRACTS, Band 115, Nr. 11, 16. September 1991 Columbus, Ohio, USA C. FROSTELL et al. "Inhaled nitric oxide: a selective pulmonary vasodilator reversing hypoxic pulmonary vasoconstriction", Seite 48, Spalten 1,2, Nr. 105 742q; & Circulation 1991, 83(6), 2038-47 (Eng.)
- CHEMICAL ABSTRACTS, Band 117, Nr. 15, 12. Oktober 1992 Columbus, Ohio, USA P.M. DUPUY et al. "Broncho- dilator action of inhaled nitric oxide in guinea pigs", Seite 55, Spalte 1, Nr. 143 200k; & J. Clin. Invest. 1992, 90(2), 421-8 (Eng.)
- CHEMICAL ABSTRACTS, Band 117, Nr. 21, 23. November 1992 Columbus, Ohio, USA J.P. KINSELLA et al. "Hemo- dynamic effects of exogenous nitric oxide in ovine transitional pulmonary circulation", Seite 556, Spalte 1, Nr. 209 592y; & Am. J. Physiol. 1992, 263(3,Pt.2), H875-H880 (Eng.)

## Beschreibung

Die Erfindung betrifft ein NO-haltiges Gasgemisch zur Behandlung pulmonaler Erkrankungen nach dem Oberbegriff des Anspruches 1.

In WO-A-92/10 228 wird der Einsatz von Stickstoffmonoxid (NO) in einem Inertgas wie Stickstoff zur Behandlung von Bronchokonstriktion oder reversibler pulmonaler Vasokonstriktion bei Mensch oder Säugetier beschrieben.

WO-A-88/06 904 beschreibt eine Methode zur Verabreichung von Inhalationsanästhetika. Es wird erwähnt, daß Helium bei schweren Fällen von Asthma eingesetzt wird.

Aus "Patent Abstracts of Japan, unexamined applications, C Field, Band 7, Nr. 61, 15. März 1983 & JP-A-57 212 121" ist im Zusammenhang mit hohen Drücken beim Tauchen zu entnehmen, daß Helium ein Bestandteil von einem Atemgas sein kann.

Die Dokumente "Chemical Abstracts, Band 115, Nr. 11, Seite 48 (No. 105 742q), 1991", "Chemical Abstracts, Band 117, Nr. 15, Seite 55 (No. 143 200k), 1992" und "Chemical Abstracts, Band 117, Nr. 21, Seite 21 (No. 209 592y), 1992" berichten über die dosisabhängige Wirkung von inhaliertem Stickstoffmonoxid beim Tier.

Aus "G. Thiele (Ed.), Handlexikon der Medizin, Seite 1022, Urban & Schwarzenberg, München 1982" ist zu entnehmen, daß Helium als Trägergas von Kunstluft für Asthmatiker und Taucher eingesetzt wird.

Nach "A. Benzing et al., Inhaliertes Stickstoffmonoxid - Anwendung und kontinuierliche Konzentrationsmessung, Anaesthesist (1993) 42: 175-178" sind nach derzeitigem Kenntnisstand 20-80 ppm NO im Inspirationsgemisch beim Menschen wirksam.

Die NO-Therapie bei Erwachsenen mit ARDS (adult respiratory distress syndrome) ist bekannt. Hierbei werden dem künstlich beatmeten Patienten über die Atemluft gegebenenfalls verhältnismäßig hohe Konzentrationen von NO aus einem mehrere 100 vpm NO enthaltenden Gasbehälter zugeführt, maximal bis etwa 60 vpm. Das NO wirkt blutdrucksenkend auf den Bluthochdruck im Lungenkreislauf, hat aber keine Wirkung auf den Blutdruck des gesamten Kreislaufes. Wegen der hohen Giftigkeit des NO und des zwangsläufig entstehenden NO₂, sowie bedingt durch die künstliche Beatmung und der daraus resultierenden kleinen kontrollierten NO-Dosiermenge, ist bei der Verabreichung des NO ein großer Überwachungsaufwand erforderlich.

Es ist erstrebenswert, die NO-Therapie auch bei solchen Patienten anzuwenden, die spontan atmen und gegebenenfalls nicht stationär behandelt werden, damit auch diese Patientengruppe von den Vorteilen der NO-Therapie profitieren kann.

Der Erfindung liegt daher die Aufgabe zugrunde, ein für diesen Zweck geeignetes NO-haltiges Gasgemisch zur Verfügung zu stellen, welches keinen großen Überwachungsaufwand erforderlich macht.

Ausgehend von dem im Oberbegriff des Anspruches 1 berücksichtigten Stand der Technik ist diese Aufgabe erfindungsgemäß gelöst mit den im kennzeichnenden Teil des Anspruches 1 angegebenen Merkmalen.

Eine vorteilhafte Weiterbildung der Erfindung ist im Unteranspruch angegeben.

Bei der praktischen Anwendung des erfindungsgemäßen Gasgemisches wird dieses mit 100 %igem medizinischen Sauerstoff so vermischt, daß ein Atemgas aus etwa 30 - 50 % O₂, Rest NO und He, entsteht. Es ist daher nur sicherzustellen, daß der O₂-Gehalt innerhalb der Grenzen von ca. 21 - 50 % eingestellt bleibt. Durch die Messung der O₂-Konzentration ist der NO-Anteil leicht einstellbar, ohne daß er meßtechnisch erfaßt werden muß. Bei patientennaher Einspeisung des Gemisches bleibt die Bildung des giftigen NO₂ äußerst gering. Gut bewährt hat sich in der praktischen Anwendung ein Gemisch mit 10 - 16 vpm NO in Helium.

Das erfindungsgemäße NO-haltige Gemisch und der medizinische O₂ können auch in zwei kleinen Speichern, z.B. Druckdosen, enthalten sein, die mit fixeingestellten Entnahmearmaturen versehen sind. Eine solche kleine, tragbare Therapieeinheit benötigt keine Steuerung und Kontrolle, so daß sie auch von einem Patienten bei sich zu Hause, beispielsweise bei einem Asthmaanfall, eingesetzt werden kann.

Das Helium in dem erfindungsgemäßen Gasgemisch hat den Vorteil, daß es wegen seiner hervorragenden Kriech- und Leichtflußeigenschaften auch in minder- oder nicht belüftete Lungenabschnitte, wie sie bei manchen Krankheitsbildern auftreten, gelangen kann. In seinem Sog zieht es NO und O₂ mit. NO bewirkt eine Erweiterung der Bronchien, wodurch diese besagten Lungenabschnitte wieder am Gasaustausch teilnehmen können.

## Patentansprüche

1. NO-haltiges Gasgemisch zur Behandlung pulmonaler Erkrankungen bei spontanatmenden Patienten, gekennzeichnet durch eine Zusammensetzung aus 2 -20 vpm NO in Helium.

2. NO-haltiges Gasgemisch nach Anspruch 1, gekennzeichnet durch 10 - 16 vpm NO in Helium.

3. Verwendung eines NO-haltigen Gasgemisches aus 2 bis 20 vpm NO in Helium zur Herstellung eines Medikamentes zur Behandlung pulmonaler Erkrankungen bei spontanatmenden Patienten.

4. Verwendung nach Anspruch 3, dadurch gekennzeichnet, daß das NO-haltige Gasgemisch 10 bis 16 vpm NO enthält.

## Claims

1. NO-containing gaseous mixture for the treatment of pulmonary diseases in spontaneously ventilating patients, characterized by a composition of 2-20 vpm of NO in helium.

2. NO-containing gaseous mixture according to Claim 1, characterized by 10-16 v-pm of NO in helium.

3. Use of an NO-containing gaseous mixture comprising 2-20 vpm of NO in helium, for preparing a medicament for the treatment of pulmonary diseases in spontaneously ventilating patients.

4. Use according to Claim 3, characterized in that the NO-containing gaseous mixture comprises 10 to 16 vpm of NO.

## Revendications

1. Mélange gazeux contenant du monoxyde d'azote (NO) pour le traitement des maladies pulmonaires chez des malades respirant spontanément,
caractérisé en ce qu'
il est composé de 2 à 20 vpm de NO dans l'hélium.

2. Mélange gazeux contenant du NO selon la revendication 1,
caractérisé par
de 10 à 16 vpm de NO dans l'hélium.

3. Utilisation d'un mélange gazeux contenant du NO constitué de 2 à 20 vpm de NO dans l'hélium pour la fabrication d'un médicament visant à traiter les maladies pulmonaires chez les malades respirant spontanément.

4. Utilisation selon la revendication 3,
caractérisée en ce que
le mélange gazeux contenant du NO contient de 10 à 16 vpm de NO.
